# EUROPEAN PATENT APPLICATION

(11) **EP 4 223 235 A1**
(43) Date of publication of application: **09.08.2023**
(21) Application number: 21888422.9
(22) Date of filing: 22.10.2021
(51) Int. Cl.: A61B 17/122, A61B 17/128

(54) **HEMOSTATIC CLIP**

(30) Priority: 06.11.2020 CN 202011232973
(71) Applicant: MicroPort Urocare (Jiaxing) Co., Ltd., Nanhu District Jiaxing Zhejiang 314006 (CN); Microport Urocare (Shanghai) Co., Ltd., Shanghai 200135 (CN)
(72) Inventor: YANG, Xuefeng, Shanghai 200135 (CN); JIANG, Lu, Shanghai 200135 (CN); WANG, Zhen, Shanghai 200135 (CN); WANG, Quanbin, Shanghai 200135 (CN)
(74) Representative: Marks & Clerk LLP
(86) International application number: PCT/CN2021/125599
(87) International publication number: WO 2022/095725

(57) **Abstract**

A hemostatic clip includes: a traction mechanism having a distal end provided with an inner bore; and a clamping mechanism including a constricting sleeve and a clip head assembly. The clip head assembly is partially disposed within the constricting sleeve and includes an engagement member and a clip head body. The engagement member has a proximal end inserted in the inner bore and forms an interference fit with the inner bore. The clip head body has a proximal end connected to a distal end of the engagement member and distally protrudes out of a distal end of the constricting sleeve. The constricting sleeve and the clip head assembly are respectively provided with a first locking member and a second locking member. When the second locking member is separate from the first locking member, the clip head body is allowed to switch between an opened configuration and a closed configuration. In the closed configuration of the clip head body, when the second locking member is connected to the first locking member, the clip head assembly is locked. Moreover, when a pulling force greater than a first predetermined value is applied to the traction mechanism, the proximal end of the engagement member will disengage from the inner bore. The separation of the engagement member from the traction mechanism does not involve the breakage of any component, thereby avoiding the risk of a broken portion remaining in a patient's body and possibly accessing a wound to cause inflammation and other issues. Thus, higher safety of use can be achieved.

## Description

### TECHNICAL FIELD

The present invention relates to medical devices and, in particular, to a hemostatic clip.

### BACKGROUND

With the continuous development of endoscopic minimally invasive surgery, various endoscopic surgical procedures, including endoscopic submucosal dissection (ESD), endoscopic retrograde cholangiopancreatography (ERCP) and natural orifice transluminal endoscopic surgery (NOTES), are widely used. For these endoscopic minimally invasive procedures, safe and effective intraoperative closure of defects or perforations in digestive tissue is a critical issue that must be addressed.

Hemostatic clips are instruments for closing defects or perforations in luminal tissue. Existing hemostatic clips have openable-and-closable and rotatably clip heads. After being delivered to a target site, such a clip head is activated by a traction mechanism to open, close and/or rotate, as desired, to clamp tissue at the target site to close a defect or perforation there. After that, the clip head is separated from the traction mechanism and remains in the patient's body until it falls off and is passed out of the body through the gastrointestinal tract after the defect or perforation is closed as a result of tissue growth at the target site.

In the existing hemostatic clips, the clip head can be coupled to the traction mechanism by a hook, and the coupling can be destroyed by breaking the hook. However, if the broken hook falls onto a wound, it may cause inflammation and other issues. Alternatively, the clip head may be coupled to the traction mechanism by a ball head and a mating connecting yoke and decoupled therefrom by causing deformation of the connecting yoke. However, the fabrication of the connecting yoke involves a complicated process.

### SUMMARY OF THE INVENTION

It is an objective of the present invention to provide a hemostatic clip, which has a simpler structure and does not have the risk of a broken portion remaining in a patient' body during its use.

To this end, the present invention provides a hemostatic clip including:
a traction mechanism defining an inner bore at a distal end thereof; and
a clamping mechanism including a constricting sleeve and a clip head assembly, the constricting sleeve provided thereon with a first locking member, the clip head assembly partially disposed within the constricting sleeve, the clip head assembly including an engagement member and a clip head body, the engagement member having a proximal end portion inserted in the inner bore so as to form an interference fit with the inner bore, the clip head body connected at a proximal end thereof to a distal end of the engagement member, the clip head body distally protruding out of a distal end of the constricting sleeve, the clip head assembly provided with a second locking member, wherein:
   the hemostatic clip is configured so that, when the second locking member is separate from the first locking member, the traction mechanism is allowed to drive, under the action of an external force, the clip head assembly to move forth and back along an axis of the constricting sleeve, thereby switching the clip head body between an opened configuration and a closed configuration; and
   the hemostatic clip is also configured so that in the closed configuration of the clip head body, movement of the clip head assembly along the axis of the constricting sleeve is prevented when the second locking member is connected to the first locking member, and the proximal end portion of the engagement member will disengage from the inner bore when a pulling force greater than a first predetermined value is applied to the traction mechanism.

Optionally, the hemostatic clip may further include an adaptor bush assembly, which is detachably connected at a distal end thereof to a proximal end of the constricting sleeve, communicates with the constricting sleeve and is configured to rotatable relative to the constricting sleeve, wherein the traction mechanism is partially disposed in the adaptor bush assembly so as to be movable along an axis thereof; and
the hemostatic clip is configured so that the traction mechanism drives, under the action of an external force, both rotation of the clip head assembly about an axis of the traction mechanism and rotation of the constricting sleeve about its own axis.

Optionally, the adaptor bush assembly may be provided with a first limiting member, wherein the traction mechanism is provided with a second limiting member, which cooperates with the first limiting member to prevent the second locking member from being connected to the first locking member.

Optionally, the adaptor bush assembly may include a sleeve, the sleeve having a distal end portion inserted in the constricting sleeve from the proximal end thereof, the sleeve also having a distal end face that makes up the first limiting member, wherein:
the traction mechanism defines at a distal end thereof first bent fins, the first bent fins bent outwardly with respect to the traction mechanism, the first bent fins configured to be elastic, the first bent fins making up the second limiting member; and
the hemostatic clip is configured so that, when the first bent fins abut against the distal end face of the sleeve, the clip head assembly is prevented from moving toward the proximal end of the constricting sleeve, and the second locking member is located distally with respect to the first locking member, and that, wherein a pulling force greater than a second predetermined value is applied to the traction mechanism, the first bent fins deforms so as to allow the traction mechanism to drive the clip head assembly to move toward the proximal end of the constricting sleeve until the second locking member is connected to the first locking member; and the second predetermined value is smaller than the first predetermined value.

Optionally, the clamping mechanism may further include a third limiting member disposed on the constricting sleeve and located distally with respect to the engagement member, the third limiting member configured to define a maximum distance that the clip head assembly is able to move toward a distal end of the constricting sleeve, thereby preventing the clip head assembly from distal dislodgement from the constricting sleeve.

Optionally, the first locking member may include a locking slot defined in a wall of the constricting sleeve, wherein the second locking member includes a stop block provided on a surface of the clip head body on a side thereof closer to an inner wall surface of the constricting sleeve; and
the third limiting member is further configured to limit circumferential relative positions of the clip head assembly and the constricting sleeve, thereby enabling the stop block to enter and engage with the locking slot.

Optionally, the third limiting member may extend radially with respect to the constricting sleeve and delimit two channels together a tubular wall of the constricting sleeve,
wherein the clip head body includes two opposing clip arms, which distally pass through the respective two channels and extend out of the constricting sleeve from the distal end thereof.

Optionally, the third limiting member may include a pin or two first stop walls provided in circumferential symmetry at the distal end of the constricting sleeve.

Optionally, the adaptor bush assembly may include a sleeve and an elastic connecting member, the sleeve including a distal section and a proximal section, the distal section having an outer diameter smaller than an outer diameter of the proximal section so that a step surface is defined on an outer wall surface of the sleeve, the distal section provided therein with a first through hole, the elastic connecting member including a base portion and a shaft portion, the base portion disposed within the sleeve and provided therein with a second through hole for passage of the traction mechanism therethrough, the shaft portion disposed on a side of the base portion closer to a distal end of the sleeve so as to extend along the axis of the sleeve, the shaft portion defining a second bent fin at a distal end thereof, the second bent fin passing through the first through hole and protruding out of the sleeve so as to define an accommodating groove together with the step surface, wherein
the constricting sleeve, at the proximal end thereof, defines an inwardly-projecting fourth limiting member, and is disposed over the distal section of the sleeve so that the fourth limiting member is disposed in the accommodating groove.

Optionally, the traction mechanism may include a core wire and a coupling tube, the coupling tube disposed at a distal end of the core wire, the coupling tube defining the inner bore, the core wire passed through the second through hole, the coupling tube located distally with respect to the base portion, the coupling tube having an outer diameter greater than a diameter of the second through hole, wherein
the hemostatic clip is configured so that, after the proximal end portion of the engagement member has disengaged from the inner bore, when the coupling tube proximally abuts against the base portion and when a pulling force greater than a third predetermined value is applied to the traction mechanism, the second bent fin will deform, thereby separating the adaptor bush assembly from the constricting sleeve.

Optionally, the hemostatic clip may further include a handle assembly, the handle assembly including a grip member, a slidable member and a rotatable member, the grip member defining an axially-extending slide slot, the slidable member disposed in the slide slot so as to be slidable therein, the rotatable member rotatably disposed at a distal end of the grip member, wherein the traction mechanism proximally extends out of the adaptor bush assembly from a proximal end thereof into the handle assembly and is connected to both the slidable member and the rotatable member in the handle assembly; and
the hemostatic clip is configured so that the slidable member drives, when sliding in the slide slot, the traction mechanism to move along the axis of the adaptor bush assembly to cause the clip head assembly to move along the axis of the constricting sleeve and that the rotatable member drives, when rotating relative to the grip member, the traction mechanism to rotate about its own axis to cause rotation of the clip head assembly and the constricting sleeve.

Optionally, the traction mechanism may include a core wire, a coupling tube, a first connecting block and a second connecting block, the coupling tube disposed at a distal end of the core wire and defining the inner bore, the first connecting block and the second connecting block both disposed over the core wire around a proximal end thereof, the second connecting block located proximally with respect to the first connecting block, the first connecting block coupled to the rotatable member and configured to be rotatable in synchronization with the rotatable member and axially movable relative to the rotatable member, the second connecting block coupled to the slidable member and configured to be axially stationary relative to the slidable member and circumferentially rotatable relative to the slidable member.

Alternatively or additionally, the adaptor bush assembly may include a spring tube distally coupled to the proximal section of the sleeve, the spring tube provided at a proximal end thereof with a locating tube, the locating tube coupled to the rotatable member in the handle assembly, the locating tube configured to be axially stationary relative to the rotatable member and circumferentially rotatable relative to the rotatable member.

The hemostatic clip of the present invention has the following advantages over the prior art:
It includes a traction mechanism and a clamping mechanism. The traction mechanism defines an inner bore at a distal end thereof. The clamping mechanism includes a constricting sleeve and a clip head assembly. The constricting sleeve is provided thereon with a first locking member, and the clip head assembly is partially disposed within the constricting sleeve. The clip head assembly includes an engagement member and a clip head body. A proximal end portion of the engagement member is inserted in the inner bore so as to form an interference fit therewith. The clip head body is connected at a proximal end thereof to a distal end of the engagement member and distally protrudes out of a distal end of the constricting sleeve. The clip head assembly is provided with a second locking member. In an initial configuration, the second locking member is separate from the first locking member. The operator may apply an external force to the traction mechanism to drive the clip head assembly to move forth and back along an axis of the constricting sleeve, thereby switching the clip head body between and opened configuration and a closed configuration. After the clip head body has clamped tissue at a target site and been switched to the closed configuration, the operator may apply a pulling force to the traction mechanism to connect the second locking member to the first locking member. As a result, the clip head body is locked in the closed configuration. Subsequently, the operator may apply a pulling force greater than a first predetermined value to the traction mechanism to disengage the proximal end portion of the engagement member from the inner bore, thus separating the clip head assembly from the traction mechanism. The interference fit between the engagement member and the inner bore enables connection of the clip head assembly and the traction mechanism to be achieved with a simple structure. Moreover, the two can be separated simply by applying a suitable pulling force, without involving the breakage of any structural component. Thus, the risk of a broken portion remaining in a patient's body and possibly accessing a wound can be avoided, resulting in higher safety and enhanced reliability.

### BRIEF DESCRIPTION OF THE DRAWINGS

The accompanying drawings are provided to facilitate a better understanding of the present invention and do not unduly limit the scope thereof in any sense, in which:
Fig. 1 is a schematic diagram showing the structure of a hemostatic clip according to an embodiment of the present invention, in which a clip head assembly is in an opened configuration;
Fig. 2 is a schematic diagram showing the structure of the hemostatic clip according to an embodiment of the present invention, in which a clip head assembly is in a closed configuration;
Fig. 3 is a schematic diagram showing the structure of a portion of a hemostatic clip according to an embodiment of the present invention;
Fig. 4 is a cross-sectional view of a portion of a hemostatic clip according to an embodiment of the present invention;
Fig. 5 is a cross-sectional view of a portion of a hemostatic clip according to an embodiment of the present invention with a clip head assembly being in a closed configuration, in which a first locking member is not connected to a second locking member;
Fig. 6 is a cross-sectional view of a portion of a hemostatic clip according to an embodiment of the present invention with a clip head assembly being in a closed configuration, in which a first locking member is connected to a second locking member and a proximal end portion of an engagement member is disengaged from an inner bore of a traction mechanism;
Fig. 7 schematically illustrates a portion of a hemostatic clip according to an embodiment of the present invention, in which a first locking member is connected to a second locking member;
Fig. 8 is a schematic diagram showing the structure of a coupling tube in a hemostatic clip according to an embodiment of the present invention
Fig. 9 is a schematic diagram showing the structure of a portion of a hemostatic clip according to an embodiment of the present invention, particularly showing the structure of a proximal end portion of a traction mechanism;
Fig. 10 is a cross-sectional view of a constricting sleeve in a hemostatic clip according to an embodiment of the present invention;
Fig. 11 schematically illustrates how a constricting sleeve mates with a third limiting member in a hemostatic clip according to an embodiment of the present invention, in which (a) is a schematic illustration before second stop walls are bent; and (b) is a schematic illustration after the second stop walls are bent to form the third limiting member;
Fig. 12 is a schematic diagram showing the structure of an engagement member in a hemostatic clip according to an embodiment of the present invention;
Fig. 13 is a cross-sectional view of a portion of a hemostatic clip according to an embodiment of the present invention, particularly showing how an adaptor bush assembly is connected to a constricting sleeve;
Fig. 14 is a schematic diagram showing the structure of an elastic connecting member in a hemostatic clip according to an embodiment of the present invention;
Fig. 15 is a schematic diagram showing the structure of a hemostatic clip according to an embodiment of the present invention; and
Fig. 16 is a schematic exploded view of a handle assembly in a hemostatic clip according to an embodiment of the present invention.

### DETAILED DESCRIPTION

Particular embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of' means "two or more", unless the context clearly dictates otherwise. As used herein, the term "or" is generally employed in the sense including "and/or" unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

Objectives, features and advantages of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear description of the embodiments. In the drawings, like reference numbers indicate identical or similar elements. Like numerals indicate like elements throughout the several views.

As used herein, the terms "proximal" and "distal" are intended to refer to relative orientations, relative positions and directions between components of a medical device or actions thereof, as viewed by a physician operating the device. Without wishing to be limiting, a "proximal end" usually refers to an end closer to the operator, and a "distal end" to an end first enters the body of a patient, during normal operation of the medical device.

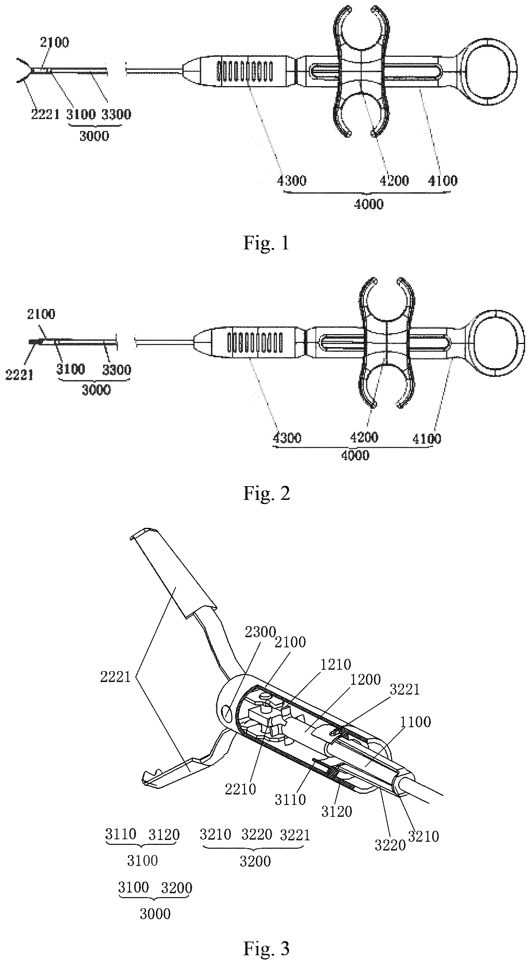

Referring to Figs. 1 to 7, in an embodiment of the present invention, there is provided a hemostatic clip including a traction mechanism 1000 and a clamping mechanism 2000. The distal end of the traction mechanism 1000 defines an inner bore 1001. The clamping mechanism 2000 includes a constricting sleeve 2100 and a clip head assembly 2200. The constricting sleeve 2100 is provided thereon with a first locking member. The clip head assembly 2200 is partially received in the constricting sleeve 2100 and includes an engagement member 2210 and a clip head body. A proximal end portion of the engagement member 2210 is inserted in the inner bore 1001 so as to form an interference fit with the inner bore 1001. The clip head body is connected to a distal end of the engagement member 2210. The distal end of the clip head body protrudes out of the distal end of the constricting sleeve 2100. The clip head assembly 2200 is also provided thereon with a second locking member.

The hemostatic clip is configured to allow, when the second locking member is separate from the first locking member, the traction mechanism to drive, under the action of an external force, the clip head assembly 2200 to move an axis of the constricting sleeve 2100 to switch the clip head body between an opened configuration and a closed configuration. In this way, the clip head body can be adjusted in position and orientation to clamp an object.

The hemostatic clip is also configured to prevent, when the clip head body is in the closed configuration and when the second locking member is connected to the first locking member, the clip head assembly 2200 from moving along the axis of the constricting sleeve 2100. In this way, when the clip head body achieves clamping of the object, the connected second and first locking members can lock the clip head body in the closed configuration, thus maintaining the clamping of the object. In this configuration, an operator may apply a pulling force greater than a first predetermined value to the traction mechanism 1000 to disengage the proximal end portion of the engagement member 2210 from the inner bore 1001, separating the clip head assembly 2200 from the traction mechanism 1000. It would be appreciated that the first predetermined value may be determined as any value depending on the strength of the interference fit established between the engagement member 2210 and the inner bore 1001, as long as the interference fit can be overcome by a pulling force exceeding the first predetermined value. In the hemostatic clip of this embodiment of the present invention, the clip head assembly 2200 is connected to the traction mechanism 1000 in a simple and reliable manner. This enables ease of use, and the two can be separated from each other without involving the breakage of any structural component, thereby avoiding the risk of a broken portion accessing a wound to cause inflammation and resulting in increased safety of use.

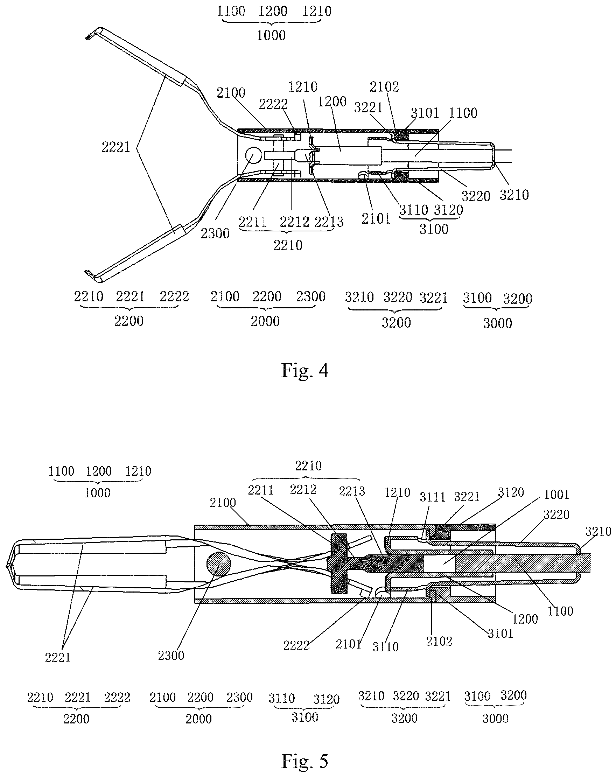

With particular reference to Figs. 4 to 6, the hemostatic clip further includes an adaptor bush assembly 3000, a distal end of the adaptor bush assembly 3000 is detachably connected to a proximal end of the constricting sleeve 2100 so as to be brought into communication with the constricting sleeve 2100. The traction mechanism 1000 is partially received in the adaptor bush assembly 3000 so as to be movable along an axis of the adaptor bush assembly 3000. By "detachably connected", it is intended to mean that the adaptor bush assembly 3000 can be detached from the constricting sleeve 2100 when a predefined condition is satisfied.

The adaptor bush assembly 3000 is provided thereon with a first limiting member, and the traction mechanism 1000 is provided thereon with a second limiting member. The second limiting member can cooperate with the first limiting member to prevent the second locking member from being connected to the first locking member. When the second locking member is not connected to the first locking member, the clip head assembly is movable forth and back along the axis of the constricting sleeve 2100 to adjust a position and orientation of the clip head assembly.

Optionally, the clamping mechanism may further include a third limiting member, the third limiting member is provided on the constricting sleeve 2100 on a distal side of the engagement member 2210 and configured to define a maximum distance that the clip head assembly 2200 is movable toward the distal end of the constricting sleeve 2100, thereby preventing the clip head assembly 2200 from distal dislodgement from the constricting sleeve 2100. Apart from this, the third limiting member is also configured to limit circumferential relative positions of the clip head assembly 2200 and the constricting sleeve 2100 to ensure circumferential alignment and hence successful connection of the second locking member with the first locking member. In this embodiment, the constricting sleeve 2100 is also configured to be rotatable relative to the adaptor bush assembly 3000. In this way, when the traction mechanism 1000 is rotated about its own axis under the action of an external force to drive the clip head assembly 2200 to rotate, the constricting sleeve 2100 will not interfere with the rotation of the clip head assembly 2200. In other words, while driving the clip head assembly 2200 to rotate about an axis of the traction mechanism 1000, the traction mechanism 1000 can simultaneously drive the constricting sleeve 2100 to rotate.

The hemostatic clip further includes a handle assembly 4000 configured to be connected to a proximal end of the traction mechanism 1000. The operator can manipulate the handle assembly 4000 to apply forces to the traction mechanism 1000, thereby activating the clamping mechanism 2000 to perform various motions.

The structures of the various components in the hemostatic clip of the present embodiment and how they are assembled together will be described in detail below with reference to the accompanying drawings. Those skilled in the art would appreciate that the structures of the components as described below are merely those suitable for use in the hemostatic clip of this embodiment rather than mandatory ones and, therefore, should not be construed as limiting the present invention in any sense.

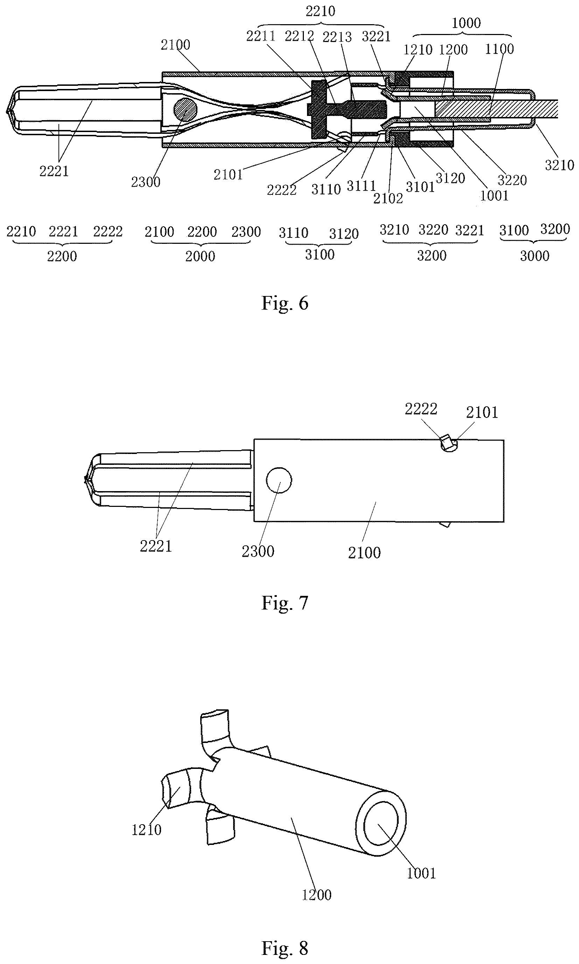

Referring to Figs. 4 to 6, in conjunction with Fig. 8, the traction mechanism 1000 includes a core wire 1100 and a coupling tube 1200. The coupling tube 1200 is disposed over a distal end portion of the core wire 1100 and defines the inner bore 1001. An outer diameter of the coupling tube 1200 is greater than an outer diameter of the core wire 1100. A distal end portion of the traction mechanism 1000, in particular, a distal end portion of the coupling tube 1200 defines a plurality of first bent fins 1210 bent outwardly with respect to the coupling tube 1200. The plurality of the first bent fin 1210 may be spaced apart, preferably, evenly, circumferentially with respect to the coupling tube 1200. Each of the first bent fins 1210 may be bent at angle of 90°. The first bent fins 1210 may be configured to be elastic.

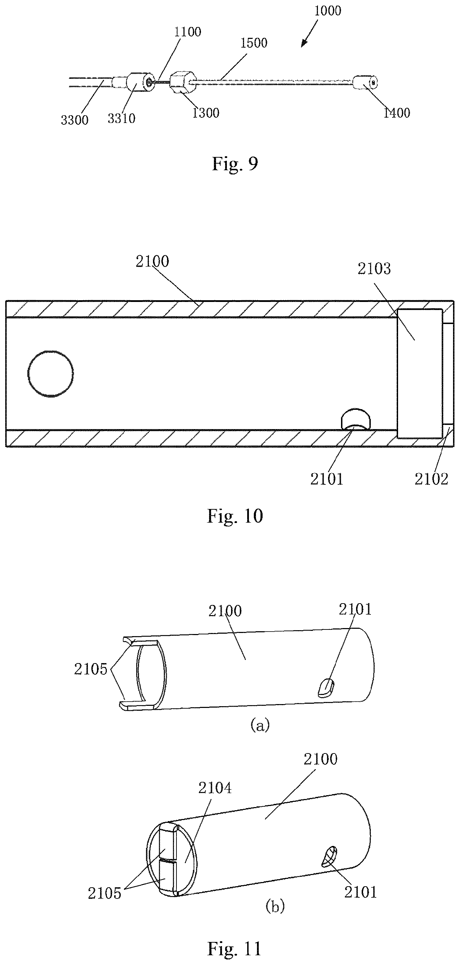

Referring to Fig. 9, the traction mechanism 1000 further includes a first connecting block 1300 and a second connecting block 1400, both first connecting block 1300 and second connecting block 1400 are located on a proximal end portion of the core wire 1100. The first connecting block 1300 is located proximally with respect to the second connecting block 1400. The first connecting block 1300 has a non-rotating shape. That is, the first connecting block has a non-circular cross-section. For example, it may have a triangular, quadrilateral, hexagonal or otherwise shaped cross-section. The second connecting block 1400 may have a circular cross-section. Both the first connecting block 1300 and the second connecting block 1400 are connected to the handle assembly 4000, as will be described in greater detail below. Preferably, the core wire 1100 includes a core wire body and a core wire jacket wrapped over an outer surface of the core wire body. The core wire jacket is provided to reduce friction that the traction mechanism 1000 encounters during its movement along the axis of the adaptor bush assembly 3000, lowering the probability of bending of the core wire 1100.

The traction mechanism 1000 further includes a reinforcing sleeve 1500 disposed over a portion of the core wire 1100 between the first connecting block 1300 and the second connecting block 1400, the opposing axial ends of the reinforcing sleeve 1500 are connected to both the first connecting block 1300 and the second connecting block 1400. The reinforcing sleeve 1500 can strengthen the traction mechanism 1000 and reduce its likelihood of bending.

Referring to Fig. 3, the constricting sleeve 2100 may be a cylindrical structure defining a first lumen axially extending therethrough. The first locking member includes locking slot 2101 defined in a wall of the constricting sleeve 2100 around the proximal end thereof. The proximal end of the constricting sleeve 2100 further defines a fourth limiting member 2102 radially projecting towards the interior of the constricting sleeve 2100 (see Fig. 6).

In some embodiments, the constricting sleeve 2100 has an inner diameter that is constant across its entire axial length. The fourth limiting member may be defined at the proximal end of the constricting sleeve 2100 in the form of a second stop wall (not shown). In some other embodiments, as shown in Fig. 10, an annular groove 2103 is defined in the inner wall surface of the constricting sleeve 2100 around the proximal end thereof. In this case, an inner wall surface portion of the constricting sleeve 2100 located proximally with respect to the annular groove 2103 serves as the fourth limiting member 2102.

Referring Figs. 3 to 7, the third limiting member is disposed at the distal end of the constricting sleeve 2100 and extends radially with respect to the constricting sleeve 2100, the third limiting member delimits two channels 2104 (see Fig. 11(b)) along with a tubular wall of the constricting sleeve 2100. In some embodiments, as shown in Figs. 3 to 7, the third limiting member includes a pin 2300, which is connected at its opposing axial ends to the tubular wall of the constricting sleeve 2100, thereby the third limiting member defining the two separate channels 2104 together with the tubular wall. In some other embodiments, as shown in Fig. 11, the third limiting member includes two first stop walls 2105 disposed on the distal end portion of the constricting sleeve 2100. The two first stop walls 2105 may be symmetrical and abut against each other. During fabrication, the two first stop walls 2105 may be formed by removing unwanted portions of a single tube. As a result, the other cylindrical portion of the tube than the first stop walls 2105 serves as the constricting sleeve 2100, while the first stop walls 2105 extend along the axis of the constricting sleeve 2100. After that, forces may be applied to the first stop walls 2105 to bend them inwardly with respect to the constricting sleeve 2100 until they extend radially with respect to the constricting sleeve 2100.

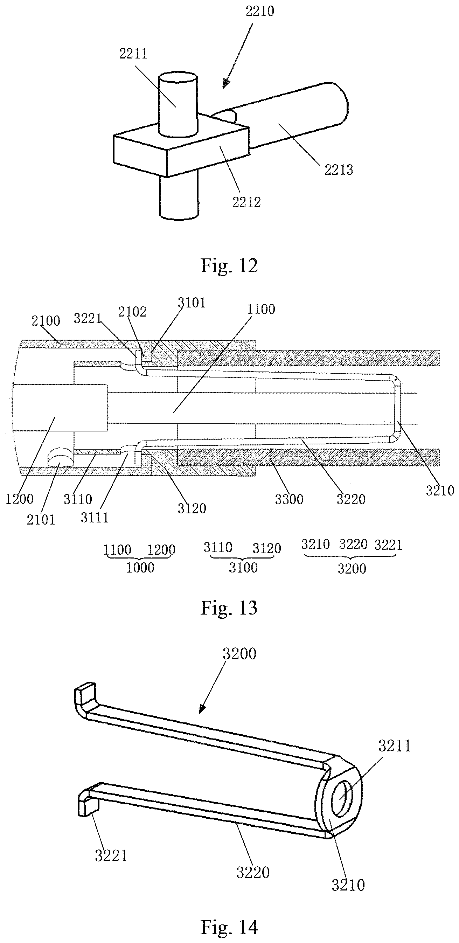

Fig. 12 is a schematic diagram showing the structure of the engagement member 2210. As shown in Fig. 12, the engagement member 2210 includes a first connecting shaft 2211, a third connecting block 2212 and a second connecting shaft 2213. The first connecting shaft 2211 and the second connecting shaft 2213 are both connected to the third connecting block 2212, and the first connecting shaft 2211 is perpendicular to the second connecting shaft 2213. Opposing axial end portions of the first connecting shaft 2211 project from the third connecting block 2212 and connected to the clip head assembly (see Figs. 4 to 6). A distal end of the second connecting shaft 2213 is connected to the third connecting block 2212, and the proximal end of the second connecting shaft 2213 is configured to be inserted into the inner bore 1001 of the traction mechanism 1000 so as to form an interference fit with the inner bore 1001 (see Figs. 4 and 5).

As shown in Figs. 4 to 6, the clip head body includes two clip arms 2221, the proximal ends of the two clip arms 2221 are connected to the respective opposing axial ends of the first connecting shaft 2211. The clip arms 2221 are configured to be elastic, the outer surfaces around the proximal ends of the clip arms 2221 are provided with the stop blocks 2222 to server as the second locking member. Here, the "outer surfaces" refer to the surfaces of the two clip arms 2221 that face away from each other, i.e., their surfaces facing the inner wall surface of the constricting sleeve 2100 when the clip arms 2221 are assembled to the constricting sleeve 2100.

When the traction mechanism 1000 and the clamping mechanism 2000 are assembled together, the distal end of the two clip arms 2221 protrude through the respective two channels 2104 out of the constricting sleeve 2100. When the operator applies a pushing force to the proximal end of the traction mechanism 1000, the traction mechanism 1000 can drive the clip head assembly 2200 to move away from the proximal end of the constricting sleeve 2100, until the tubular wall of the constricting sleeve 2100 does not compress the clip arms 2221 any longer. As a result, the two clip arms 2221 moves away from each other, switching the clip head body to the opened configuration. When the operator applies a pulling force to the proximal end of the traction mechanism 1000, the traction mechanism 1000 can drive the clip head assembly 2200 to move toward the proximal end of the constricting sleeve 2100. In this process, the tubular wall of the constricting sleeve 2100 will compress the clip arms 2221 to cause them to move toward each other, thereby switching the clip head body to the closed configuration. It would be appreciated that, constrained by the third limiting member (e.g., the pin 2300, or the first stop walls 2105), the clip head assembly 2200 can move substantially only along the axis of the constricting sleeve 2100 without circumferential rotation (such circumferential rotation is made impossible, for example, because the portions of the clip arms 2221 located distally with respect to the constricting sleeve 2100 abut against the tubular wall of the constricting sleeve 2100 and the third limiting member).

Upon the stop blocks 2222 reaching the locking slots 2101 as a result of the movement of the clip head assembly 2200, the stop blocks 2222 will enter and engage with the locking slots 2101 (see Fig. 7). As a result, the clip head assembly 2200 is locked and cannot further move axially relative to the constricting sleeve 2100. Subsequently, the operator may apply a pulling force to the proximal end of the traction mechanism 1000, which is greater than the first predetermined value and can overcome the interference fit between the proximal end portion of the engagement member 2210 (in particular, of the second connecting shaft 2213) and the inner bore 1001. As a result, the second connecting shaft 2213 can be disengaged from the inner bore 1001 (see Fig. 6), separating the engagement member 2210 from the traction mechanism 1000. It would be appreciated that the "pushing force" refers to a force acting in the direction from the proximal to distal end of the traction mechanism 1000 and the "pulling force" refers to a force acting in the direction from the distal to proximal end of the traction mechanism 1000.

Referring back to Figs. 4 to 6, in conjunction with Figs. 13 and 14, the adaptor bush assembly 3000 includes a sleeve 3100 and an elastic connecting member 3200. The distal end of the sleeve 3100 is inserted in the first lumen of the constricting sleeve 2100 from the proximal end of the constricting sleeve 2100. Specifically, the sleeve 3100 includes a distal section 3110 and a proximal section 3120, which are joined to each other. An outer diameter of the distal section 3110 is smaller than an outer diameter of the proximal section 3120 so that a step surface 3101 is defined on an outer wall surface of the sleeve 3100 and that the distal section 3110 can be inserted into the first lumen from the proximal end of the constricting sleeve 2100. The distal section 3110 is provided therein with first through holes 3111. The elastic connecting member 3200 includes a base portion 3210 and shaft portions 3220. The base portion 3210 is disposed within a second lumen defined by the sleeve 3100 and defines a second through hole 3211 therein. The second through hole 3211 has a diameter, which is greater than or equal to the outer diameter of the core wire 1100 and smaller than the outer diameter of the coupling tube 1200 so that the base portion 3210 can be disposed over the core wire 1100. The shaft portions 3220 are provided on the side of the base portion 3210 closer to a distal end of the sleeve 3100. The shaft portions 3220 extends along an axis of the sleeve 3100. The distal end of the shaft portions 3220 defines second bent fins 3221, each of the second bent fins 3221 is outwardly bent at an angle of 90°-120° (i.e., an angle between the specific second bent fin 3221 and the respective shaft portion 3220). The second bent fins 3221 (radially) pass through the first through holes 3111 and protrude out of the sleeve 3100 so as to define, along with the step surface 3101 of the sleeve 3100, a stop groove for cooperating with the fourth limiting member of the constricting sleeve 2100, thereby connecting the constricting sleeve 2100 to the adaptor bush assembly 3000 (i.e., when the constricting sleeve 2100 is connected to the adaptor bush assembly 3000, the fourth limiting member is confined between the second bent fins 3221 and the step surface 3101). When the second bent fins 3221 deform under the action of an external force so that the angles between the second bent fins 3221 and the shaft portions 3220 increase to an appropriate value, the second bent fins 3221 will disengage from the first through holes 3111 inside the adaptor bush assembly 3000, separating the constricting sleeve 2100 from the adaptor bush assembly 3000.

With particular reference to Fig. 5, during movement of the engagement member 2210 and the clip head assembly toward the proximal end of the constricting sleeve 2100 as a result of movement of the traction mechanism 1000 toward a proximal end of the adaptor bush assembly 3000 along the axis of the adaptor bush assembly 3000 under the action of a pulling force, before the stop blocks 2222 in the clip head assembly reach the locking slots 2101 in the constricting sleeve 2100, the first bent fins 1210 of the coupling tube 1200 abut against a distal end face of the adaptor bush assembly 3000 (more precisely, of the sleeve 3100), preventing the traction mechanism 1000 from further moving toward the proximal end of the adaptor bush assembly 3000 and thus preventing the stop blocks 2222 from engaging with the locking slots 2101. That is to say, the distal end face of the adaptor bush assembly 3000 provides the first limiting member, and the first bent fins 1210 provide the second limiting member. When a pulling force greater than a second predetermined value is applied to the traction mechanism 1000, the first bent fins 1210 will deform, increasing the angles between the first bent fins 1210 and an outer surface of the coupling tube 1200, until the distal end portion of the adaptor bush assembly 3000 does not limit the first bent fins 1210 anymore. As a result, the traction mechanism 1000 can further move toward the proximal end of the adaptor bush assembly 3000, allowing the stop blocks 2222 to reach the locking slots 2101 in the constricting sleeve 2100. The second predetermined value may be determined as actually needed, but is generally smaller than the first predetermined value.

Further, referring back to Fig. 13, in conjunction with Fig. 9, the adaptor bush assembly 3000 further includes a spring tube 3300, a distal end of the spring tube 3300 is connected to a proximal end of the sleeve 3100 (i.e., the proximal end of the proximal section 3120) and a proximal end of the spring tube 3300 is provided with a locating tube 3310. The locating tube 3310 may have a circular cross-section and is configured for connection with the handle assembly 4000.

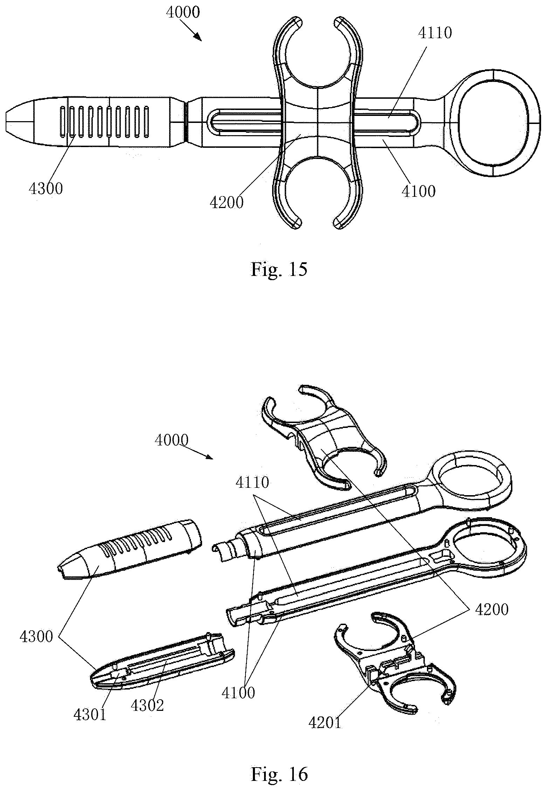

Further, referring to Fig. 15, the handle assembly 4000 includes a grip member 4100, a slidable member 4200 and a rotatable member 4300. The grip member 4100 defines an axially-extending slide slot 4110, and the slidable member 4200 is coupled to the slide slot 4110 so as to be slidable within the slide slot 4110. The rotatable member 4300 is rotatably disposed at a distal end of the grip member 4100. Referring to Figs. 1 and 2, a proximal end of the traction mechanism 1000 (more precisely, the proximal end of the core wire 1100) extends out of the adaptor bush assembly 3000 from the proximal end thereof (so that the first connecting block 1300 is located proximally with respect to the locating tube 3310) and is coupled to both the slidable member 4200 and the rotatable member 4300 in the handle assembly 4000. Accordingly, the hemostatic clip is configured so that the slidable member 4200 drives, when sliding in the slide slot 4110, the slidable member 4200 and hence the traction mechanism 1000 to move along the axis of the adaptor bush assembly 3000. Moreover, the rotatable member 4300 drives, when rotating relative to the grip member 4100, the rotatable member 4300 and hence the traction mechanism 1000 to rotate.

With additional reference to Figs. 10 and 16, the rotatable member 4300 defines therein a first securing cavity 4301 and a second securing cavity 4302, and the slidable member 4200 defines therein a third securing cavity 4201. The first securing cavity 4301, the second securing cavity 4302 and the third securing cavity 4201 communicate with one another. The first securing cavity 4301 and the third securing cavity 4201 may have circular cross-sections, but the present invention is not so limited. For example, the third securing cavity 4201 may alternatively have a rectangular cross-section. The second securing cavity 4302 may have a cross-section matching that of the first connecting block 1300. The locating tube 3310 of the spring tube 3300 is disposed in the first securing cavity 4301 and configured to be axially stationary relative to the rotatable member 4300. Moreover, the rotatable member 4300 is configured to be rotatable relative to the locating tube 3310, avoiding twisting of the adaptor bush assembly 3000 during its rotation with the traction mechanism 1000. The first connecting block 1300 of the traction mechanism 1000 is disposed in the second securing cavity 4302 and configured to be axially movable within the second securing cavity 4302 and rotatable in synchronization with the rotatable member 4300. The second locating block 1400 of the traction mechanism 1000 is disposed in the third securing cavity 4201 and configured to be axially stationary relative to the slidable member 4200 and circumferentially rotatable relative to the slidable member 4200, thereby avoiding twisting of the traction mechanism 1000 during its rotation.

In this embodiment, the grip member 4100, the slidable member 4200 and the rotatable member 4300 are all preferred to be modular structures, which can facilitate assembly of the handle assembly 4000 with the traction mechanism 1000 and the adaptor bush assembly 3000.

A method of using the hemostatic clip will be described below.

After the hemostatic clip is delivered to a target site in a patient's body, an operator may manipulate the handle assembly 4000 to cause the slidable member 4200 to slide (distally) within the slide slot 4110 to drive the traction mechanism 1000 to move along the axis of the adaptor bush assembly 3000. As a result, the clip head assembly is driven to move (distally) along the axis of the constricting sleeve 2100, thereby switching the clip head body to the opened configuration. Moreover, the operator may manipulate the rotatable member 4300 to cause the traction mechanism 1000 and hence the clip head assembly to rotate to adjust a position and orientation of the clip head assembly until tissue at the target site is located between the two clip arms 2221. In this way, a pulling force applied by the operator to the slidable member 4200 results in a pulling force smaller than the second predetermined value being exerted on the traction mechanism 1000. Thus, the distal end face of the adaptor bush assembly 3000 stops the first bent fins 1210, preventing the stop blocks 2222 from coming into engagement with the locking slots 2101. As a result, the operator is allowed to make repeated positional and orientational adjustments to the clip head assembly.

After that, the operator may proximally pull the slidable member 4200 to cause the traction mechanism 1000 to drive the clip head assembly to move toward the proximal end of the constricting sleeve 2100 until the first bent fins 1210 comes into abutment against the distal end face of the adaptor bush assembly 3000 (i.e., the first limiting member).

Subsequently, the operator may increase the pulling force to a value greater than the second predetermined value to cause deformation of the first bent fins 1210.

Afterward, the operator may further proximally retract the slidable member 4200 until the stop blocks 2222 enter the locking slots 2101.

Next, the operator may increase the pulling force to a value greater than the first predetermined value to overcome the interference fit between the engagement member 2210 and the inner bore 1001, separating the engagement member 2210 from the traction mechanism 1000.

Following that, the operator may further proximally pull the slidable member 4200 until the coupling tube 1200 comes into abutment against the base portion 3210 of the elastic connecting member 3200. The operator may then increase the pulling force to a value greater than a third predetermined value. When transmitted by the coupling tube 1200 to the elastic connecting member 3200, the pulling force will cause the second bent fins 3221 deform and disengage from the first through holes 3111, separating the adaptor bush assembly 3000 from the constricting sleeve 2100. The third predetermined value may be determined as needed.

Finally, the operator may additionally pull the slidable member 4200 proximally to withdraw the adaptor bush assembly 3000 and the traction mechanism 1000 from the patient's body.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A hemostatic clip, comprising:
a traction mechanism, a distal end of the traction mechanism defining an inner bore; and
a clamping mechanism comprising a constricting sleeve and a clip head assembly, the constricting sleeve provided thereon with a first locking member, the clip head assembly partially disposed within the constricting sleeve, the clip head assembly comprising an engagement member and a clip head body, the engagement member having a proximal end inserted in the inner bore and forming an interference fit with the inner bore, a proximal end of the clip head body connected to a distal end of the engagement member, a distal end of the clip head body protruding out of a distal end of the constricting sleeve, the clip head assembly provided with a second locking member, wherein:
the hemostatic clip is configured so that, when the second locking member is separate from the first locking member, the traction mechanism is allowed to drive, under the action of an external force, the clip head assembly to move forth and back along an axis of the constricting sleeve, thereby switching the clip head body between an opened configuration and a closed configuration; and
the hemostatic clip is also configured so that in the closed configuration of the clip head body, movement of the clip head assembly along the axis of the constricting sleeve is prevented when the second locking member is connected to the first locking member, and the proximal end of the engagement member will disengage from the inner bore when a pulling force greater than a first predetermined value is applied to the traction mechanism.

2. The hemostatic clip of claim 1, further comprising an adaptor bush assembly, a distal end of the adaptor bush assembly detachably connected to a proximal end of the constricting sleeve, communicates with the constricting sleeve and is configured to rotatable relative to the constricting sleeve, wherein the traction mechanism is partially disposed in the adaptor bush assembly and is movable along an axis of the adaptor bush assembly; and
the hemostatic clip is configured so that the traction mechanism is able to drive, under the action of an external force, both rotation of the clip head assembly about an axis of the traction mechanism and rotation of the constricting sleeve about its own axis.

3. The hemostatic clip of claim 2, wherein the adaptor bush assembly is provided with a first limiting member, wherein the traction mechanism is provided with a second limiting member, the second limiting member cooperating with the first limiting member to prevent the second locking member from being connected to the first locking member.

4. The hemostatic clip of claim 3, wherein the adaptor bush assembly comprises a sleeve, a distal end of the sleeve inserted in an interior of the constricting sleeve from the proximal end of the constricting sleeve, a distal end face of the sleeve making up the first limiting member, wherein:
a distal end of the traction mechanism defines first bent fins, the first bent fins bent outwardly with respect to the traction mechanism, the first bent fins configured to be elastic, the first bent fins making up the second limiting member; and
the hemostatic clip is configured so that, when the first bent fins abut against the distal end face of the sleeve, the clip head assembly is prevented from moving toward the proximal end of the constricting sleeve, and the second locking member is located distally with respect to the first locking member, wherein a pulling force greater than a second predetermined value is applied to the traction mechanism, the first bent fins deforms so as to allow the traction mechanism to drive the clip head assembly to move toward the proximal end of the constricting sleeve until the second locking member is connected to the first locking member; and the second predetermined value is smaller than the first predetermined value.

5. The hemostatic clip of any one of claims 1 to 4, wherein the clamping mechanism further comprises a third limiting member disposed on the constricting sleeve and located distally with respect to the engagement member, the third limiting member configured to define a maximum distance that the clip head assembly is able to move toward the distal end of the constricting sleeve, thereby preventing the clip head assembly from distal dislodgement from the constricting sleeve.

6. The hemostatic clip of claim 5, wherein the first locking member comprises a locking slot defined in a wall of the constricting sleeve, wherein the second locking member comprises a stop block provided on a surface of the clip head body on a side thereof closer to an inner wall surface of the constricting sleeve; and
the third limiting member is further configured to limit circumferential relative positions of the clip head assembly and the constricting sleeve, thereby enabling the stop block to enter and engage with the locking slot.

7. The hemostatic clip of claim 6, wherein the third limiting member extends radially with respect to the constricting sleeve and delimits two channels together a tubular wall of the constricting sleeve,
wherein the clip head body comprises two opposing clip arms, distal ends of the clip arms passing through the respective two channels and extending out of the distal end of the constricting sleeve.

8. The hemostatic clip of claim 7, wherein the third limiting member comprises a pin; or wherein the third limiting member comprises two first stop walls provided at the distal end of the constricting sleeve, the two first stop walls arranged symmetrically with each other.

9. The hemostatic clip of claim 2, wherein the adaptor bush assembly comprises a sleeve and an elastic connecting member, the sleeve comprising a distal section and a proximal section, the distal section having an outer diameter smaller than an outer diameter of the proximal section so that a step surface is defined on an outer wall surface of the sleeve, the distal section provided therein with a first through hole, the elastic connecting member comprising a base portion and a shaft portion, the base portion disposed within the sleeve and provided therein with a second through hole for passage of the traction mechanism therethrough, the shaft portion disposed on a side of the base portion closer to a distal end of the sleeve so as to extend along the axis of the sleeve, a distal end of the shaft portion defining a second bent fin, the second bent fin passing through the first through hole and protruding out of the sleeve so as to define an accommodating groove together with the step surface,
the proximal end of the constricting sleeve defining an inwardly-projecting fourth limiting member, the proximal end of the constricting sleeve disposed over the distal section of the sleeve, and the fourth limiting member disposed in the accommodating groove.

10. The hemostatic clip of claim 9, wherein the traction mechanism comprises a core wire and a coupling tube, the coupling tube disposed at a distal end of the core wire, the coupling tube defining the inner bore, the core wire passing through the second through hole, the coupling tube located distally with respect to the base portion, the coupling tube having an outer diameter greater than a diameter of the second through hole,
wherein the hemostatic clip is configured so that, after the proximal end of the engagement member has disengaged from the inner bore, when the coupling tube proximally abuts against the base portion and when a pulling force greater than a third predetermined value is applied to the traction mechanism, the second bent fin will deform, thereby separating the adaptor bush assembly from the constricting sleeve.

11. The hemostatic clip of claim 9, further comprising a handle assembly, the handle assembly comprising a grip member, a slidable member and a rotatable member, the grip member defining an axially-extending slide slot, wherein the slidable member is disposed in the slide slot and is slidable in the slide slot, the rotatable member rotatably disposed at a distal end of the grip member, wherein the proximal end of the traction mechanism extends out of a proximal end of the adaptor bush assembly into the handle assembly, thereby connecting to both the slidable member and the rotatable member of the handle assembly; and
the hemostatic clip is configured so that the slidable member drives, when sliding in the slide slot, the traction mechanism to move along the axis of the adaptor bush assembly to cause the clip head assembly to move along the axis of the constricting sleeve and that the rotatable member drives, when rotating relative to the grip member, the traction mechanism to rotate about its own axis to cause rotation of the clip head assembly and the constricting sleeve.

12. The hemostatic clip of claim 11, wherein the traction mechanism comprises a core wire, a coupling tube, a first connecting block and a second connecting block, the coupling tube disposed at a distal end of the core wire and defining the inner bore, the first connecting block and the second connecting block both disposed over a proximal end of the core wire, the second connecting block located proximally with respect to the first connecting block, the first connecting block coupled to the rotatable member and configured to be rotatable in synchronization with the rotatable member and axially movable relative to the rotatable member, the second connecting block coupled to the slidable member and configured to be axially stationary relative to the slidable member and circumferentially rotatable relative to the slidable member, and/or
the adaptor bush assembly comprises a spring tube distally coupled to the proximal section of the sleeve, a proximal end of the spring tube provided with a locating tube, the locating tube coupled to the rotatable member in the handle assembly, the locating tube configured to be axially stationary relative to the rotatable member and circumferentially rotatable relative to the rotatable member.
